(19) Europäisches Patentamt / European Patent Office / Office européen des brevets

(11) **EP 4 162 806 A1**

(12) **EUROPEAN PATENT APPLICATION**
published in accordance with Art. 153(4) EPC

(43) Date of publication:
**12.04.2023 Bulletin 2023/15**

(21) Application number: **21821861.8**

(22) Date of filing: **07.06.2021**

(51) International Patent Classification (IPC):
**A23J 3/14** (2006.01)    **A23J 3/16** (2006.01)
**A61P 3/02** (2006.01)    **A23L 5/00** (2016.01)
**A23L 33/185** (2016.01)    **A61K 38/02** (2006.01)
**C12P 21/00** (2006.01)    **A23L 2/00** (2006.01)
**A23L 2/66** (2006.01)

(52) Cooperative Patent Classification (CPC):
**A23J 3/14; A23J 3/16; A23L 2/00; A23L 2/66;**
**A23L 5/00; A23L 33/185; A61K 38/02; A61P 3/02;**
C12P 21/00

(86) International application number:
**PCT/JP2021/021605**

(87) International publication number:
**WO 2021/251344 (16.12.2021 Gazette 2021/50)**

(84) Designated Contracting States:
**AL AT BE BG CH CY CZ DE DK EE ES FI FR GB**
**GR HR HU IE IS IT LI LT LU LV MC MK MT NL NO**
**PL PT RO RS SE SI SK SM TR**
Designated Extension States:
**BA ME**
Designated Validation States:
**KH MA MD TN**

(30) Priority: **08.06.2020 JP 2020099538**

(71) Applicants:
• **Amano Enzyme Inc.**
**Nagoya-shi**
**Aichi 460-8630 (JP)**

• **Amano Enzyme Europe Ltd.**
**Oxfordshire, OX75SR (GB)**

(72) Inventor: **FUJIOKA, Hiroki**
**Chipping Norton, Oxfordshire, OX75SR (GB)**

(74) Representative: **Müller-Boré & Partner**
**Patentanwälte PartG mbB**
**Friedenheimer Brücke 21**
**80639 München (DE)**

(54) **IMPROVING TEXTURE OF PROTEIN**

(57)    The present invention addresses the problem of providing a method for improving a texture of a protein. By allowing a protein deamidase to act on a substrate protein, a feeling of the protein on a tongue can be smoothened, and the texture thereof can be improved.

EP 4 162 806 A1

## Description

TECHNICAL FIELD

The present invention relates to an improvement in a texture of a protein. The present invention is particularly useful for improving a texture of a vegetable protein or a food or beverage containing a vegetable protein.

BACKGROUND ART

Against a background of allergy problems, an increase in the number of vegetarians, religious reasons, and the like, proteins of plant origin such as soybeans, grain, and nuts have come into widespread use as alternative materials for foods or beverages using a milk protein source derived from animals, typified by milk.

On the other hand, in the case of replacing a milk protein material with a plant-derived protein material, since a type and functionality of a protein, or components constituting flavor and taste are different, improvement in the texture, taste, and flavor is required. For example, there is known a method for obtaining an odorless and palatable protein by allowing lipase to act on a soybean protein and then removing the resulting hydrolysate (Patent Document 1). In addition, there is known a method in which a rotary blade shearing force is applied to a swollen soybean to refine the swollen soybean to an average particle diameter of 100 microns or less, and then a frictional shearing force is applied to refine the swollen soybean to obtain soy milk having a smooth good mouth feel (Patent Document 2).

In evaluating improvement in physical properties of a food, sensory evaluation is performed in which a texture is evaluated by actually tasting the food, but it is difficult to accurately evaluate the texture by sensory evaluation since sensations caused by various organs in the mouth such as feeling on the tongue, feeling on the teeth, and feeling in the mouth occur during a time between chewing and swallowing after putting a food into the mouth. It is also known that physical properties are changed due to mixing of the food with saliva during chewing.

Viscosity measurement and hardness measurement are used as methods for representing a texture evaluation by a physical index, but the texture during chewing is a result of a physical influence of a food on various organs in the mouth, and it is difficult to evaluate the texture during chewing only by these methods. Therefore, various research and development on methods for evaluating a texture are currently performed. As one of them, in recent years, a method for evaluating a texture in which tribology is applied has also been developed and studied. (Non-Patent Document 1)

PRIOR ART DOCUMENTS

PATENT DOCUMENTS

Patent Document 1: Japanese Patent Laid-open Publication No. H06-30710
Patent Document 2: Japanese Patent Laid-open Publication No. H10-295308

NON-PATENT DOCUMENT

Non-Patent Document 1: Campbell CL et al. J Texture Stud. 2017 48 p 335-341

SUMMARY OF THE INVENTION

PROBLEMS TO BE SOLVED BY THE INVENTION

In general, when glutamine and asparagine residues in a protein are deamidated to generate a carboxyl group, a negative charge of the protein increases, and as a result, an isoelectric point decreases and a hydration force increases. Furthermore, deamidation of glutamine and asparagine residues in the protein leads to a decrease in interaction between proteins due to an increase in electrostatic repulsion, that is, a decrease in an associative property. These changes greatly increase solubility and water dispersibility of the protein. In addition, the increase in the negative charge of the protein loosens folding of the protein, changes a higher-order structure, and exposes a hydrophobic region buried inside the molecule to a surface of the molecule. Therefore, the deamidated protein has amphiphilicity and becomes an ideal surfactant, and emulsifying power, emulsifying stability, a foaming property, and foam stability of the protein are greatly improved. As described above, it is known that deamidation of a protein leads to improvement in various functional properties of the protein, and that use of the protein is dramatically increased (e.g., Molecular Approaches to Improving Food Quality and Safety, D. Chatnagar and T. E. Cleveland, eds., Van Nostrand Reinhold, New York, 1992, p.37). In addition, it is also known that when an arginine residue in a protein is deiminated, hydrophobicity of the protein is increased to change a higher-order structure of the protein. However, it has not been conventionally known that when a protein deamidase is allowed to act on a protein, a feeling of the protein on the tongue when the protein is eaten becomes smooth and a texture of the protein is improved.

MEANS FOR SOLVING THE PROBLEMS

In order to solve the above problems, the present inventor conducted studies using a vegetable protein beverage (an oat beverage, soy milk). As a result, it has been found that physical properties of a protein are changed by an action of a protein deamidase, and a coefficient of friction generated between the protein and the tongue is reduced, so that a feeling on the tongue becomes smooth. In other words, it has been revealed that when a protein deamidase is allowed to act on a protein, a feeling on the tongue becomes smooth and a texture is improved. Based on this finding, the present inventor has completed the following inventions.

[1] A method for improving a texture of a protein, the method including allowing a protein deamidase to act on the protein.
[2] The method for improving a texture of a protein according to [1], wherein the protein deamidase is an enzyme that deamidates an amide group of a glutamine residue in the protein.
[3] The method for improving a texture of a protein according to [2], wherein the protein deamidase is a protein glutaminase.
[4] The method for improving a texture of a protein according to any one of [1] to [3], wherein the protein is a vegetable protein.
[5] The method for improving a texture according to [4], wherein the vegetable protein is a vegetable protein in a vegetable protein solution.
[6] The method for improving a texture of a protein according to [4] or [5], wherein the vegetable protein is a soybean protein or an oat protein.
[7] The method for improving a texture of a protein according to any one of [1] to [6], the method being performed to smooth a feeling of the protein on a tongue.
[8] An agent for improving a texture of a protein containing a protein deamidase in a protein.
[9] A protein having a smooth feeling on a tongue due to an action of a protein deamidase on the protein.
[10] A food or beverage or a pharmaceutical product containing the protein according to [9].

EMBODIMENTS OF THE INVENTION

1. Method for improving texture of protein

The method for improving a texture of the present invention is characterized by allowing a protein deamidase to act on a protein. Hereinafter, the method for improving a texture of a protein in the present invention will be described in more detail.

A type, an origin, and the like of the protein deamidase that can be used in the method for improving a texture of the present invention are not particularly limited. The origin is, for example, animal-derived, plant-derived, or microorganism-derived. In addition, in the case of an enzyme derived from a microorganism, the enzyme may be accumulated in either inside or outside a cell body of the microorganism. Furthermore, the enzyme may be only by those existing in nature but also those produced by a genetic engineering technique and a cell engineering technique. Alternatively, the enzyme may be an enzyme protein modified by a protein engineering technique. Furthermore, as the protein deamidase (e.g., protein glutaminase), it is desirable to use a purified enzyme having a high purity, but the purity is not limited as long as a desired reaction is possible. In addition, an enzyme preparation may be used as the protein deamidase, and in this case, various salts, saccharides, proteins, lipids, surfactants, and the like may be added as enzyme stabilizers to the enzyme preparation.

The protein deamidase used in the method for improving a texture of the present invention includes an enzyme that deamidates an amide group of a glutamine residue and/or an asparagine residue in a protein, and an enzyme that deiminates an arginine residue in a protein.

Examples of the enzyme that deamidates a glutamine residue in a protein include a protein glutaminase. The protein glutaminase is an enzyme that hydrolyzes an amide group of a side chain of a glutamine residue in a protein to convert the glutamine residue into a glutamic acid residue. Examples of the protein glutaminase include, but are not limited to, a protein glutaminase derived from Chryseobacterium proteolyticum (Eur J Biochem, 268 (5), 1410, 2001, Protein-glutaminase From Chryseobacterium Proteolyticum, an Enzyme That Deamidates Glutaminyl Residues in Proteins. Purification, Characterization and Gene Cloning, S Yamaguchi 1, D J Jeenes, D B Archer, or Front Microbiol, 9, 1975, 2018, Complete Genome Sequence and Characterization of a Protein-Glutaminase Producing Strain, Chryseobacterium proteolyticum QSH1265, Ruidan Qu, Xiaoyu Zhu, Min Tian, Yingjie Liu, Wenjuan Yang, Jian Ye, Hongliang Gao, Jing Huang).

Examples of the enzyme that deamidates an asparagine residue in a protein include, but are not limited to, the enzyme disclosed in WO 2015/133590.

Furthermore, examples of the enzyme that deiminates an arginine residue include, but are not limited to, an arginine deiminase derived from Fusarium graminearum.

In the method for improving a texture of the present invention, the protein on which the protein deamidase is allowed to act (hereinafter, it may be referred to as "substrate protein") may be any protein that is used as a food material and whose texture is desired to be improved, and there are no particular restrictions on the origin, properties, and the like, but examples thereof include vegetable proteins, insect-derived proteins, microorganism-derived proteins, algae-derived proteins, and animal proteins.

Examples of the vegetable protein include proteins derived from beans such as soybeans, green peas, lentils, chickpeas, and black beans; proteins derived from cereals such as wheat, barley, oats, and rice; proteins derived from nuts such as almonds and peanuts; and proteins derived from seeds such as hemp seeds, chia seeds, quinoa, and Amaranthus. Examples of the insect protein include proteins derived from crickets. Examples of the microorganism-derived protein include proteins derived from yeasts, proteins derived from filamentous fungi, and proteins derived from fungi called mycoproteins of mushrooms. Examples of the algae-derived protein include Spirulina. Examples of the animal protein include milk proteins such as casein and β-lactoglobulin; egg protein such as ovalbumin; meat proteins such as myosin and actin; blood proteins such as serum albumin; and tendon proteins such as gelatin and collagen.

In addition, the substrate protein may be a chemically partially degraded protein by an acid, an alkali, or the like, an enzymatically partially degraded protein by a protease or the like, a chemically modified protein by various reagents, a synthetic peptide, or the like.

In the method for improving a texture of the present invention, the substrate protein is subjected to a reaction in a solution, slurry, or paste state, but a concentration thereof is not particularly limited, and the concentration may be determined according to a desired property and state. In addition, not limited to an aqueous solution, a solution in a form of an emulsion with oil and fat, a slurry, or a paste may be subjected to a reaction, and a salt, a saccharide, a protein, a flavor, a humectant, a colorant, or the like may be added to the solution, the slurry, or the paste of the substrate protein as necessary. Preferably, a vegetable protein solution is subjected to a reaction. In other words, the protein deamidase acts on the substrate protein (vegetable protein) present in the vegetable protein solution. Examples of the vegetable protein solution include a soybean protein solution such as soy milk and an oat protein solution such as oat milk. A concentration of the substrate protein in the solution, slurry, or paste containing the substrate protein is not particularly limited, but the lower limit is, for example, 0.1% (w/v), preferably 0.15% (w/v) or 0.2% (w/v), more preferably 0.2% (w/v) or 0.5% (w/v), still more preferably 0.5% (w/v) or 1% (w/v), and even more preferably 2% (w/v), and the upper limit is, for example, 30% (w/v), preferably 20% (w/v), more preferably 10% (w/v), still more preferably 8% (w/v), and even more preferably 5% (w/v). Specific examples of the concentration of the substrate protein in the solution, slurry or paste containing the substrate protein include 0.1 to 30% (w/v), preferably 0.15 to 20% (w/v), more preferably 0.2 to 10% (w/v), still more preferably 0.5 to 8% (w/v), and even more preferably 2 to 5% (w/v). As used herein, "% (w/v)" has the same meaning as "g/100 ml".

In the method for improving a texture of the present invention, an amount of an enzyme, a reaction time, a temperature, a pH of a reaction solution, and the like to be used are not particularly limited. Usually, the amount of an enzyme is 0.01 to 100,000 U, preferably 0.1 to 50,000 U, and more preferably 1 to 10,000 U of the protein deamidase per g of the substrate protein. The reaction temperature is 5 to 80°C, preferably 20 to 60°C. The pH of the reaction solution is 2 to 10, preferably 4 to 8. The reaction time is 10 seconds to 48 hours, preferably 10 minutes to 24 hours. A texture of a protein is improved by the above reaction conditions. These reaction conditions are appropriately selected according to a type, an existence state, and the like of the substrate protein. Optimum reaction conditions may be determined through a preliminary experiment. In the case of a protein glutaminase, an activity value of the protein deamidase is a value measured according to <Method for measuring activity of protein glutaminase> described below. In the case of an enzyme that deamidates an asparagine residue, the activity value is a value measured by changing the substrate to Z-Asn-Gly-OH (N-benzyloxycarbonyl-L-asparagyl-glycine) by the method described in <Method for measuring activity of protein glutaminase> described below.

In a protein obtained by the method for improving a texture of the present invention, a coefficient of friction generated between the protein and the tongue when the protein is mixed with saliva in the mouth is reduced, so that a feeling on the tongue becomes smooth. According to the present invention, a texture or palatableness of a protein is improved as a result of a feeling on the tongue becoming smooth. In the protein obtained by the method of the present invention, that is, a protein that has been treated with the protein deamidase, typically, a coefficient of friction measured under the following conditions is reduced to 90% or less (e.g., 70 to 90%), preferably 85% or less (e.g., 70 to 85%), as compared with a case of using a protein without being treated with the protein deamidase.

<Method of measuring coefficient of friction>

A sample solution is prepared by adding artificial saliva (solution at pH 6.7 containing 50 U/ml $\alpha$-amylase, 25.7 mM NaCl, 8.6 mM $K_2HPO_4$, 4.7 mM $Na_2CO_3$, and 2% (w/w) mucin) to a concentration of 15% (v/v) to a solution in which protein is added to water to a concentration of 0.2 g/100 mL, and a coefficient of friction is measured by tribology measurement. Specifically, with 200 $\mu$L of the sample solution interposed between an artificial tongue (made of silicone resin) and an artificial palate (made of polydimethylsiloxane), the artificial tongue is slid on the artificial palate at a load of 0.5 N and a slide speed of 35 mm/s, and a coefficient of friction on the artificial tongue is measured.

2. Agent for improving texture

The present invention further provides an agent for improving a texture of a protein containing a protein deamidase. As used herein, the "agent for improving a texture of a protein " is an agent used for improving a texture of a protein by smoothing a feeling of the protein on the tongue.

The texture improver of the present invention is an enzyme used in the method for improving a texture, and a mode of use or the like thereof is as described in the section of "1. Method for improving texture of protein" above.

3. Method for producing texture-improved protein, or food or beverage or pharmaceutical product containing texture-improved protein

By using the method for improving a texture, it is possible to produce a texture-improved protein (i.e., a protein having a smooth feeling on the tongue due to an action of a protein deamidase), or a food or beverage or a pharmaceutical product containing the same. One embodiment of the method for producing a texture-improved protein includes the following steps (1) and (2). After step (2), a step of deactivating a protein deamidase (step (3)) may be added.

(1) A step of preparing a protein on which a protein deamidase is allowed to act.
(2) A step of treating the prepared protein with a protein deamidase.

On the other hand, one embodiment of the method for producing a food or beverage or a pharmaceutical product includes the following steps (I) and (II).

(I) A step of preparing a food material, a beverage material, or a pharmaceutical material containing a protein on which a protein deamidase is allowed to act.
(II) A step of treating the prepared food material, beverage material, or pharmaceutical material with a protein deamidase.

The treated material obtained by step (II) is subjected to further treatment (e.g., heat treatment, sterilization treatment, mixing with other raw materials, or addition to a food or beverage) as necessary, and a final product, that is, a product containing a protein having an improved texture as a result of a feeling on the tongue becoming smooth is obtained.

A type of the food or beverage containing the texture-improved protein is not particularly limited, and examples thereof include noodles and breads (various noodles, white breads, pastries, and the like), processed cereals (cereals, oatmeal, muesli, processed rice, wheat bran, barley tea, and the like), confectionery and desserts (biscuits, baked confectionery, rice confectionery, oil confectionery, Japanese confectionery, Western confectionery, semi-dried confectionery, Japanese dry confectionery, candies, chocolates, chewing gums, snacks, jellies, frozen confectionery, and the like), vegetable milk (milk containing various nuts such as almonds, cashew nuts, hazelnuts, pecan nuts, macadamia nuts, pistachios, walnuts, brazil nuts, peanuts, coconuts, chestnuts, sesame as a raw material, and milk containing pine nuts, soybeans, oats, peas, hemp, lupin beans, broad beans, chick beans, barley, wheat, rice, Japanese barnyard millet, foxtail millet, canary seed, teff, quinoa, or linseed as a raw material), soups, tofu, meat substitutes and dairy substitutes containing vegetable materials (cheeses substitutes, fermented milk substitutes), roux (curry roux, stew roux, and the like), nutritional supplements and beverages (protein powders, protein beverages, supplements, nutritional drinks, and the like), foods for pets, and nutritional supplements for pets.

Among these foods or beverages, vegetable milk is preferable from the viewpoint that smoothness of a feeling on the tongue is more easily felt and a more excellent effect of improving a texture can be exhibited. In order to produce the vegetable milk containing the texture-improved protein, specifically, vegetable milk may be prepared in the step (I), and the vegetable milk may be treated with a protein deamidase in the step (II).

EXAMPLES

Hereinafter, the present invention will be further described with reference to Examples.

In the following Examples, activity of a protein glutaminase was measured by the method described below using Z-Gln-Gly-OH (N-benzyloxycarbonyl-L-glutaminyl-glycine) as a substrate unless otherwise specified.

<Method for measuring activity of protein glutaminase>

To 100 $\mu$l of a 176 mmol/l phosphate buffer solution (pH 6.5) containing 10 mmol/l Z-Gln-Gly-OH, 10 $\mu$l of an enzyme solution is added, and the mixture is incubated at 37°C for 60 minutes. Then, 100 $\mu$l of a 12% trichloroacetic acid solution is added to stop the reaction. After centrifugation (15,000 rpm, 4°C, 5 minutes), the supernatant is measured using F-kit ammonia (manufactured by Boehringer Mannheim GmbH) as follows (A1). Separately, measurement is performed in the same manner using water instead of the enzyme solution (A2). To 100 $\mu$l of F-kit ammonia reagent 2, 10$\mu$l of the supernatant and 190 $\mu$l of water are added, the mixture is allowed to stand at room temperature for 5 minutes, and then absorbance (E1) at 340 nm is measured using 100 $\mu$l of the mixture. After 1.0 $\mu$l of reagent 3 (glutamate dehydrogenase) is added to the remaining 200 $\mu$l, the mixture is further allowed to stand at room temperature for 20 minutes, and then absorbance (E2) at 340 nm of the remaining 200 $\mu$l is measured. An amount of an enzyme liberating 1 $\mu$mol of ammonia per minute under the above conditions is defined as 1 unit, and the activity is determined according to the following formula.

$$U/ml = 1.76 \times [A1(E1 - E2) - A2(E1 - E2)]$$

<Example 1>

An effect of a protein glutaminase (PG) on improving a texture of a protein was examined using an oat beverage (raw materials: water, oat, chicory root fibre, sunflower oil, calcium, sea salt, gellan gum, vitamins, protein content 0.2 g/100 mL, Alpro). To the oat beverage, a protein glutaminase (product name: protein glutaminase "Amano" 500, manufactured by Amano Enzyme Inc.) was added in an amount of 5 U per g of a substrate protein, and after mixing, a reaction was caused at 50°C for 30 minutes. The reaction was terminated by treatment at 95°C for 10 minutes. The enzyme-treated (PG-treated) oat beverage was subjected to sensory evaluation and measurement of a coefficient of friction. The coefficient of friction was measured by tribology measurement in which a sample was placed on an artificial tongue and slid at a specific speed. Specifically, with 200 $\mu$L of the enzyme-treated oat beverage to which artificial saliva (solution at pH 6.7 containing 50 U/ml $\alpha$-amylase, 25.7 mM NaCl, 8.6 mM $K_2HPO_4$, 4.7 mM $Na_2CO_3$, and 2% (w/w) mucin; manufactured by Sigma-Aldrich Co. LLC) was added so as to have a concentration of 15% (v/v) interposed between an artificial tongue (made of silicone resin) and an artificial palate (made of polydimethylsiloxane), the artificial tongue was slid on the artificial palate at a load of 0.5 N to measure the coefficient of friction on the artificial tongue. An oat beverage not subjected to enzyme treatment (PG treatment) was also measured in the same manner. A ratio (%) of the coefficient of friction of the sample with PG treatment to the coefficient of friction (COF) of the sample without PG treatment was calculated for each slide speed, and an effect of PG treatment was evaluated.

The results are shown in Table 1.

[Table 1]

| Slide speed (mm/s) | Coefficient of friction (COF) | | Ratio (to without PG treatment) |
|---|---|---|---|
| | Without PG treatment | With PG treatment | |
| 7 | 0.40 | 0.37 | 92% |
| 18 | 0.39 | 0.32 | 84% |
| 35 | 0.36 | 0.30 | 83% |
| 53 | 0.33 | 0.29 | 86% |
| 70 | 0.31 | 0.28 | 91% |

As shown in Table 1, it was found that by treating the oat beverage with the protein glutaminase, the coefficient of friction with the artificial tongue in the presence of the artificial saliva was significantly reduced. Also in the sensory evaluation, it was confirmed that a feeling on the tongue became smooth when treated with protein glutaminase.

<Example 2>

Evaluation was performed in the same manner as in Example 1, except that the protein raw material used was changed to soy milk (raw materials: water, hulled soya beans protein content 3.0 g/100 mL, Alpro).

The results are shown in Table 2.

[Table 2]

| Slide speed (mm/s) | Coefficient of friction (COF) | | Ratio (to without PG treatment) |
|---|---|---|---|
| | Without PG treatment | With PG treatment | |
| 7 | 0.49 | 0.43 | 87% |
| 18 | 0.46 | 0.39 | 84% |
| 35 | 0.42 | 0.34 | 81% |
| 53 | 0.39 | 0.31 | 79% |
| 70 | 0.36 | 0.28 | 76% |

As shown in Table 2, it was found that by treating the soy milk with the protein glutaminase, the coefficient of friction with the artificial tongue in the presence of the artificial saliva was significantly reduced. Also in the sensory evaluation, it was confirmed that a feeling on the tongue became smooth when treated with protein glutaminase.

INDUSTRIAL APPLICABILITY

According to the method for improving a texture of the present invention, a coefficient of friction in the mouth is reduced, and a protein having a smooth feeling on the tongue can be obtained. The protein obtained by the present invention is used, for example, in the field of food processing such as dairy substitutes (cheeses substitutes, fermented milk substitutes) using vegetable materials or vegetable milk. Furthermore, as a novel protein-derived material, the protein can also be used as a material for medical supplies and supplements.

The present invention is not limited to the description of the embodiments of the invention and Examples above. Various modifications that can be easily conceived by those skilled in the art without departing from the scope of the claims are also included in the present invention. The entire contents of the articles, published patent publications, patent publications, and the like specified in this specification are incorporated herein by reference.

**Claims**

1.  A method for improving a texture of a protein, the method comprising allowing a protein deamidase to act on the protein.

2.  The method for improving a texture of a protein according to claim 1, wherein the protein deamidase is an enzyme that deamidates an amide group of a glutamine residue in the protein.

3.  The method for improving a texture of a protein according to claim 2, wherein the protein deamidase is a protein glutaminase.

4.  The method for improving a texture of a protein according to any one of claims 1 to 3, wherein the protein is a vegetable protein.

5.  The method for improving a texture according to claim 4, wherein the vegetable protein is a vegetable protein in a vegetable protein solution.

6.  The method for improving a texture of a protein according to claim 4 or 5, wherein the vegetable protein is a soybean protein or an oat protein.

7.  The method for improving a texture of a protein according to any one of claims 1 to 6, the method being performed to smooth a feeling of the protein on a tongue.

8. An agent for improving a texture of a protein comprising a protein deamidase in a protein.

9. A protein comprising a smooth feeling on a tongue due to an action of a protein deamidase on the protein.

10. A food or beverage or a pharmaceutical product comprising the protein according to claim 9.

## INTERNATIONAL SEARCH REPORT

International application No.

PCT/JP2021/021605

### A. CLASSIFICATION OF SUBJECT MATTER

A23J 3/14(2006.01)i; A23J 3/16(2006.01)i; A61P 3/02(2006.01)i; A23L 5/00(2016.01)i; A23L 33/185(2016.01)i; A61K 38/02(2006.01)i; C12P 21/00(2006.01)n; A23L 2/00(2006.01)i; A23L 2/66(2006.01)i
FI:      A23J3/14; A23J3/16; A23L2/00 B; A23L2/00 J; A23L33/185; A23L5/00 Z; A61K38/02; A61P3/02; C12P21/00 Z

According to International Patent Classification (IPC) or to both national classification and IPC

### B. FIELDS SEARCHED

Minimum documentation searched (classification system followed by classification symbols)
A23J3/14; A23J3/16; A61P3/02; A23L5/00; A23L33/185; A61K38/02; C12P21/00; A23L2/00; A23L2/66

Documentation searched other than minimum documentation to the extent that such documents are included in the fields searched

| | |
|---|---|
| Published examined utility model applications of Japan | 1922–1996 |
| Published unexamined utility model applications of Japan | 1971–2021 |
| Registered utility model specifications of Japan | 1996–2021 |
| Published registered utility model applications of Japan | 1994–2021 |

Electronic data base consulted during the international search (name of data base and, where practicable, search terms used)
JSTPlus/JMEDPlus/JST7580 (JDreamIII); JSTChina (JDreamIII)

### C. DOCUMENTS CONSIDERED TO BE RELEVANT

| Category* | Citation of document, with indication, where appropriate, of the relevant passages | Relevant to claim No. |
|---|---|---|
| X | WO 2009/113628 A1 (AJINOMOTO CO., INC.) 17 September 2009 (2009-09-17) claims, example 4 | 1–10 |
| X | WO 2009/113627 A1 (AJINOMOTO CO., INC.) 17 September 2009 (2009-09-17) examples | 1–5, 7–10 |
| X | WO 2006/075771 A1 (AJINOMOTO CO., INC.) 20 July 2006 (2006-07-20) examples | 1–3, 7–10 |
| X | WO 2009/154212 A1 (AJINOMOTO CO., INC.) 23 December 2009 (2009-12-23) examples | 1–3, 7–10 |
| X | WO 2006/075772 A1 (AJINOMOTO CO., INC.) 20 July 2006 (2006-07-20) examples | 1–3, 7–10 |

☒ Further documents are listed in the continuation of Box C.  ☒ See patent family annex.

| | | | |
|---|---|---|---|
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| 16 July 2021 (16.07.2021) | 27 July 2021 (27.07.2021) |

| Name and mailing address of the ISA/ | Authorized officer |
|---|---|
| Japan Patent Office 3-4-3, Kasumigaseki, Chiyoda-ku, Tokyo 100-8915, Japan | |
| | Telephone No. |

Form PCT/ISA/210 (second sheet) (January 2015)

**INTERNATIONAL SEARCH REPORT**

| International application No. |
| --- |
| PCT/JP2021/021605 |

C (Continuation).   DOCUMENTS CONSIDERED TO BE RELEVANT

| Category* | Citation of document, with indication, where appropriate, of the relevant passages | Relevant to claim No. |
| --- | --- | --- |
| X | JP 2015-524276 A (NESTEC S.A.) 24 August 2015 (2015-08-24) claims, paragraphs [0001], [0008], examples | 10 |

Form PCT/ISA/210 (continuation of second sheet) (January 2015)

<table>
<tr><td colspan="2" align="center"><strong>INTERNATIONAL SEARCH REPORT</strong><br>Information on patent family members</td><td>International application No.<br><br>PCT/JP2021/021605</td></tr>
</table>

| Patent Documents referred in the Report | Publication Date | Patent Family | Publication Date |
|---|---|---|---|
| WO 2009/113628 A1 | 17 Sep. 2009 | US 2011/0064847 A1 claims, example 4 EP 2267146 A1 CN 102016057 A KR 10-2010-0127824 A | |
| WO 2009/113627 A1 | 17 Sep. 2009 | (Family: none) | |
| WO 2006/075771 A1 | 20 Jul. 2006 | US 2007/0254066 A1 examples EP 1836907 A1 KR 10-2007-0104528 A CN 101090644 A | |
| WO 2009/154212 A1 | 23 Dec. 2009 | US 2011/0151055 A1 examples EP 2305047 A1 | |
| WO 2006/075772 A1 | 20 Jul. 2006 | US 2007/0254065 A1 examples EP 1839491 A1 CN 101098626 A | |
| JP 2015-524276 A | 24 Aug. 2015 | US 2015/0257403 A1 claims, paragraphs [0001], [0009], examples EP 2882297 A1 CN 104540388 A | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP H0630710 A
- JP H10295308 A

- WO 2015133590 A

**Non-patent literature cited in the description**

- **CAMPBELL CL et al.** *J Texture Stud.,* 2017, vol. 48, 335-341
- Molecular Approaches to Improving Food Quality and Safety. Van Nostrand Reinhold, 1992, 37
- **S YAMAGUCHI 1 ; D J JEENES ; D B ARCHER.** Protein-glutaminase From Chryseobacterium Proteolyticum, an Enzyme That Deamidates Glutaminyl Residues in Proteins. Purification, Characterization and Gene Cloning. *Eur J Biochem,* 2001, vol. 268 (5), 1410

- **RUIDAN QU ; XIAOYU ZHU ; MIN TIAN ; YINGJIE LIU ; WENJUAN YANG ; JIAN YE ; HONGLIANG GAO ; JING HUANG.** Complete Genome Sequence and Characterization of a Protein-Glutaminase Producing Strain, Chryseobacterium proteolyticum QSH1265. *Front Microbiol,* 2018, vol. 9, 1975